# EUROPEAN PATENT APPLICATION

(11) **EP 4 438 036 A1**
(43) Date of publication of application: **02.10.2024**
(21) Application number: 22914974.5
(22) Date of filing: 28.12.2022
(51) Int. Cl.: A61K 9/20, A61K 31/415, A61P 35/00, A61P 29/00

(54) **SOLID PHARMACEUTICAL COMPOSITION**

(30) Priority: 30.12.2021 CN 202111661891
(71) Applicant: Adlai Nortye Biopharma Co., Ltd., Yuhang District Hangzhou Zhejiang 311121 (CN)
(72) Inventor: CHEN, Liang, Hangzhou, Zhejiang 311121 (CN); LIU, Shifeng, Hangzhou, Zhejiang 311121 (CN); ZHAO, Yanhui, Hangzhou, Zhejiang 311121 (CN); YANG, Donghui, Hangzhou, Zhejiang 311121 (CN); LU, Yang, Hangzhou, Zhejiang 311121 (CN)
(74) Representative: Michalski Hüttermann & Partner Patentanwälte mbB
(86) International application number: PCT/CN2022/143030
(87) International publication number: WO 2023/125724

(57) **Abstract**

The present invention provides a solid pharmaceutical composition, which comprises a compound represented by formula 1 or a pharmaceutically acceptable salt thereof and a pharmaceutically acceptable carrier, wherein the compound represented by formula 1 or a pharmaceutically acceptable salt thereof comprises from about 30% to about 80% of the total weight of the solid pharmaceutical composition.

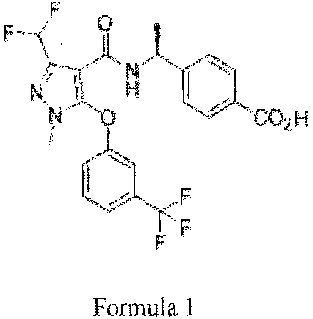

## Description

The present application claims the priority of Chinese patent application No.202111661891.1, filed on December 30, 2021, which is hereby incorporated by reference in its entirety.

### TECHNICAL FIELD

The invention belongs to the technical field of medicine, in particular to a solid pharmaceutical composition of novel selective EP4 antagonist 4-{(1S)-1-[({3-(difluoromethyl)-1-methyl-5-[3-(trifluoromethyl)phenoxy]-4-1H-pyrazolyl} carbonyl)amino]ethyl}benzoic acid and optionally at least one pharmaceutically acceptable carrier, and methods for their preparation.

### BACKGROUND

The most enriched immune cells infiltrating solid tumors include myeloid cells, mainly TAMs and MDSCs, and in animal models, depletion of monocytic lineages, including TAMs and MDSCs, significantly inhibits tumor growth in established tumors (Zeisberger, et al., 2006; Srivastava, et al., 2012). The literature has well documented the presence and accumulation of TAMs and MDSCs in tumors, which are associated with poorer prognosis in patients with breast, pancreatic, or other types of cancer (Joyce and Pollard, 2009; Ding, et al., 2009; Ganjoo, et al., 2011; Medrek, et al., 2012; Zhang, et al., 2012).

Both TAMs and MDSCs are derived from immature monocyte precursors. EP4, one of four PGE2 receptors, is expressed on myeloid cells (Wang and DuBois, 2010) and plays a key role in promoting monocyte differentiation into TAMs and MDSCs in the tumor microenvironment (Lechner, et al., 2011; Mao, et al., 2014; Majumder, et al., 2014). Therefore, EP4 antagonism is a potential anticancer therapy, and the mechanism of action is to attenuate the formation and function of immunosuppressive TAMs and MDSCs.

The structure of 4-{(1S)-1-[({3-(difluoromethyl)-1-methyl-5-[3-(trifluoromethyl)phenoxy]-4-1H-pyrazolyl} carbonyl)amino]ethyl}benzoic acid is shown in formula 1, which is abbreviated as AN0025 hereinafter in this application. It is a highly selective and potent antagonist of prostaglandin E2 (PGE2) receptor 4 (EP4) (PGE2-EP4), which exhibits antitumor activity by regulating the accumulation and function of immunosuppressive myelocytes (including tumor-associated macrophages [TAM] and myelogenic suppressor cells [MDSC]) in tumor microenvironment.

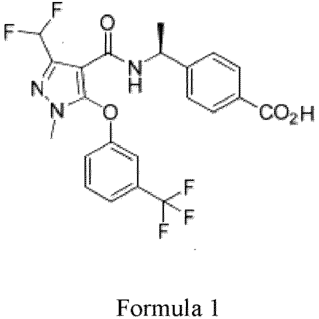

Oral administration is a more convenient and safer route of administration, superior to other routes of administration (such as intramuscular injection, intravenous injection, etc.), and is the preferred method of administration during drug development.

AN0025 capsules, which were developed for Phase I clinical studies, involve AN0025 and a pharmaceutically acceptable carrier, prepared by dry granulation technology to obtain granules containing the active ingredient AN0025, which are then filled into hypromellose hollow capsule. Additional details of this capsule are described below as "Comparative Example 1".

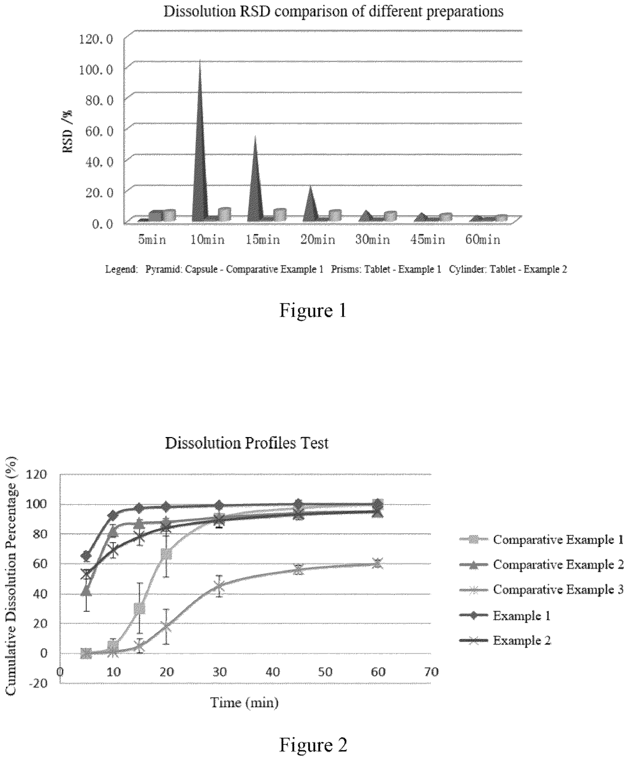

However, the above-mentioned intra-batch variation (RSD) of AN0025 capsules is relatively large (Figure 1), resulting in unreliable dissolution test results, and it is difficult to establish a good quality evaluation method by means of dissolution to evaluate the impact of different material variables and process variables on product quality. It is also difficult to establish good quality control methods to ensure process robustness and quality consistency of AN0025 formulations between batches. Therefore, there is a need to develop suitable and robust solid pharmaceutical compositions to overcome the aforementioned problems of large dissolution variability.

### SUMMARY OF THE INVENTION

The present invention relates to a quality controllable solid dosage form and a process for its preparation, wherein the solid dosage form comprises a compound of formula 1 4-{(1S)-1-[({3-(difluoromethyl)-1-methyl-5-[3-(trifluoromethyl)phenoxy]-4-1H-pyrazolyl} carbonyl)amino]ethyl}benzoic acid (compound AN0025) and optionally at least one pharmaceutically acceptable carrier. AN0025 belongs to the class IV drug of the Biopharmaceutical Classification System, that is, low solubility and poor permeability. The inventor unexpectedly found in the experiment that AN0025 tablets prepared by different processes can show good uniformity in in vitro dissolution experiments, and AN0025 tablets prepared with different API particle sizes have similar in vitro dissolution behavior and in vivo exposure. The bioavailability of the AN0025 tablet provided by the present invention in beagle dogs is comparable to that of the clinical capsule, but the individual differences in in vivo exposure (AUC) are small. These results indicate that AN0025 tablet has better controllability in commercial production and better efficacy and safety in clinical application.

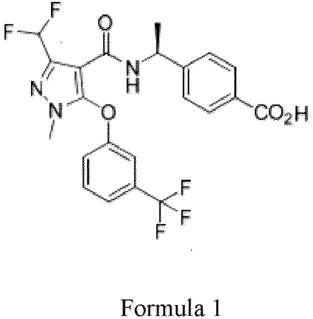

In one aspect, the present invention provides a solid pharmaceutical composition, which comprises: the compound described in formula 1 or a pharmaceutically acceptable salt thereof and a pharmaceutically acceptable carrier, wherein the compound described in formula 1 or a pharmaceutically acceptable salt thereof comprises from about 30% to about 80% of the total weight of the solid pharmaceutical composition; the solid pharmaceutical composition is in the form of a tablet.

In one embodiment, the compound of formula 1, or a pharmaceutically acceptable salt thereof, comprises from about 40% to about 60% of the total weight of the solid pharmaceutical composition.

In one embodiment, the pharmaceutically acceptable carrier includes one or more selected from fillers, binders, lubricants, disintegrants and glidants.

In one embodiment, the pharmaceutically acceptable carrier comprises:
a. a filler, which comprises from about 5% to about 60% of the total weight of the solid pharmaceutical composition;
b. a binder, which comprises from about 0% to about 8% of the total weight of the solid pharmaceutical composition;
c. a disintegrant, which comprises from about 0% to about 30% of the total weight of the solid pharmaceutical composition;
d. a lubricant, which comprises from about 0.1% to about 10% of the total weight of the solid pharmaceutical composition;
e. a glidant, which comprises from about 0% to about 5% of the total weight of the solid pharmaceutical composition.

In yet another embodiment, the pharmaceutically acceptable carrier comprises:
a. a filler, which comprises from about 10% to about 50% of the total weight of the solid pharmaceutical composition;
b. a binder, which comprises from about 2% to about 6% of the total weight of the solid pharmaceutical composition;
c. a disintegrant, which comprises from about 0.1% to about 25% of the total weight of the solid pharmaceutical composition;
d. a lubricant, which comprises from about 0.5% to about 8% of the total weight of the solid pharmaceutical composition;
e. a glidant, which comprises from about 0% to about 3% of the total weight of the solid pharmaceutical composition.

In a preferred embodiment, the pharmaceutically acceptable carrier comprises:
a. a filler, which comprises from about 20% to about 40% of the total weight of the solid pharmaceutical composition;
b. a binder, comprises from about 3% to about 5% of the total weight of the solid pharmaceutical composition;
c. a disintegrant, which comprises from about 1% to about 25% of the total weight of the solid pharmaceutical composition;
d. a lubricant, which comprises from about 1% to about 6% of the total weight of the solid pharmaceutical composition;
e. a glidant, which comprises from about 0% to about 1% of the total weight of the solid pharmaceutical composition.

In one embodiment, the filler is selected from the group consisting of microcrystalline cellulose, calcium phosphate, calcium hydrogen phosphate, starch, dextrin, pregelatinized starch, mannitol, sorbitol, lactose, dextran, sucrose, dextrose and combinations thereof; the binder is selected from the group consisting of cellulose ethers, povidone, copovidone, starch, corn syrup and combinations thereof; wherein the cellulose ethers include but are not limited to hypromellose, hydroxypropyl cellulose, methyl cellulose, hydroxyethyl cellulose; the disintegrant is selected from the group consisting of sodium croscarmellose, crospovidone, sodium carboxymethyl starch, carboxymethylcellulose calcium, low-substituted hydroxypropyl cellulose and combinations thereof; the lubricant is selected from the group consisting of magnesium stearate, sodium stearyl fumarate, polyethylene glycol, glycerin, behenate, talc and combinations thereof.

In one embodiment, the filler is selected from microcrystalline cellulose, lactose, mannitol, sorbitol and combinations thereof; the binder is selected from hydroxypropyl cellulose, hypromellose, povidone, copovidone and combinations thereof; the disintegrant is selected from sodium croscarmellose, crospovidone, sodium carboxymethyl starch, carboxymethylcellulose calcium, low-substituted hydroxypropyl cellulose and combinations thereof; the lubricant is selected from magnesium stearate, sodium stearyl fumarate and combinations thereof.

In one embodiment, the solid pharmaceutical composition of the present invention includes granules having an internal phase.

In one embodiment, the solid pharmaceutical composition of the present invention further comprises an outer phase adjacent to the granules having an inner phase.

In one embodiment, for the solid pharmaceutical composition provided by the present invention, AN0025 and other pharmaceutically acceptable carriers are prepared into granules with good fluidity and compressibility through a granulation process, and then compressed into tablets. All other pharmaceutically acceptable carriers in the solid pharmaceutical composition may be added internally to participate in the granulation process, or may be partially added as an external phase.

In one embodiment, the granules having an internal phase are prepared by a dry preparation process, comprising the steps of: screening and weighing Compound AN0025 and each pharmaceutically acceptable carrier; blending Compound AN0025 and a pharmaceutically acceptable carrier for an appropriate amount of time; rolling the blend into strips and grinding the strips into granules; blend the granules with extragranular excipients for an appropriate amount of time; compressing the blend into tablets.

In one embodiment, the granules having an internal phase are prepared by a wet preparation process, comprising the steps of: screening and weighing Compound AN0025 and each pharmaceutically acceptable carrier; while adding the granulation fluid with or without a binder, mix Compound AN0025 and a pharmaceutically acceptable carrier for an appropriate amount of time at a suitable mixing speed and chop the mixture into granules, wherein the granulation fluid can be a binder solution prepared by dispersing a binder in a solvent (water), or it can be just a wetting agent, such as water; drying the granules; blending the granules with extragranular excipients for an appropriate amount of time; compressing the blend into tablets .

In one embodiment, the solid pharmaceutical composition further comprises a coating.

In one embodiment, the compound of formula 1 has a particle size with a D90 of less than about 200 microns.

In a preferred embodiment, the compound of formula 1 has a particle size with a D90 of less than about 100 microns.

In one embodiment, the solid pharmaceutical composition of the present invention contains from about 50 mg to about 500 mg of the compound of formula 1.

In a preferred embodiment, the solid pharmaceutical composition of the present invention contains from about 100 mg to about 300 mg of the compound of formula 1.

In another aspect, the present invention provides the use of the solid pharmaceutical composition for preparing a medicament for the treatment of multiple sclerosis, rheumatoid arthritis, or cancer, and the cancer includes: skin cancer, breast cancer, colorectal cancer, prostate cancer, kidney cancer, ovarian cancer, cervical cancer, endometrial cancer, glioblastoma, lung cancer, head and neck cancer, medulloblastoma and urethral cancer.

### BRIEF DESCRIPTION OF THE DRAWINGS

Figure 1 Comparison of dissolution RSD of Comparative Example 1, Example 1, Example 2
Figure 2 Comparison of dissolution profiles of Comparative Example 1, 2, 3 and Example 1, 2
Figure 3 AN0025 drug concentration-time curve of capsule (Comparative Example 1) in beagle dogs (750mg/dog)
Figure 4 AN0025 drug concentration-time curve of tablet (Example 4) in beagle dogs (750mg/dog)
Figure 5 AN0025 drug concentration-time curve of capsule (Comparative Example 1) in beagle dogs (250mg/dog)
Figure 6 AN0025 drug concentration-time curve of tablet (Example 4) in beagle dogs (250mg/dog)
Figure 7 AN0025 drug concentration-time curve of tablet (Example 4a) in beagle dogs (750mg/dog)

### EMBODIMENTS

### Definitions

The present invention provides a quality-controllable solid pharmaceutical composition, a solid pharmaceutical preparation, and a preparation process thereof, wherein the quality-controllable solid pharmaceutical composition comprises a compound 4-{(1S)-1-[({3-(difluoromethyl)-1-methyl-5-[3-(trifluoromethyl)phenoxy]-4-1H-pyrazolyl}carbo nyl)amino]ethyl}benzoic acid (AN0025) and pharmaceutically acceptable salts thereof, and optionally at least one pharmaceutically acceptable carrier.

Unless otherwise specified, the following terms as used herein have the following meanings: The term "pharmaceutical composition" or "preparation" refers to a physical mixture containing a therapeutic compound to be administered to an individual (eg, a human) to prevent, treat or manage a particular disease or condition affecting the individual. For example, the terms "pharmaceutical composition" or "preparation" as used herein also encompass intimate physical mixtures formed under high temperature and high pressure.

The term "pharmaceutically acceptable" refers to compounds, materials, compositions, carriers and/or dosage forms that, within the scope of sound medical judgment, are suitable for contact with the tissues of an individual (especially a human) without undue toxicity, irritation, allergic reactions and other problematic complications and with a reasonable benefit/risk ratio.

The terms "pharmaceutically acceptable carrier" and "carrier" refer to any pharmaceutically acceptable inert material that is substantially biologically inactive and that forms a substantial part of a preparation.

The term "therapeutically effective amount" refers to an amount or concentration effective to reduce, eliminate, treat, prevent or control the symptoms of a disease or condition affecting an individual. The term "control" refers to all processes in which the progression of diseases and conditions affecting mammals can be slowed, interrupted, arrested or stopped. However, "control" does not necessarily indicate complete elimination of all symptoms of the disease and condition.

The term "treatment" includes prophylactic and/or therapeutic treatment as well as delaying the progression of a disease or disorder. Preferably, the term "treatment" refers to therapeutic treatment as well as delaying the progression of a disease or disorder. "Delayed progression" refers to the administration of a pharmaceutical composition to individuals in a pre- or early stage of the cancer to be treated, in these patients, for example, diagnosing a pre-formation of the corresponding disease, or where the individual is in a situation where the corresponding disease is likely to develop.

The term "oral dosage form" refers to a pharmaceutical composition prepared for administration to an individual by the oral route of administration. Examples of known oral dosage forms include, but are not limited to, tablets, capsules, caplets, powders, pills, granules, solutions, suspensions, solution and solution preconcentrates, emulsions and emulsion preconcentrates, and the like. In some aspects, powders, pills, granules, and tablets can be coated with suitable polymers or commonly used coating materials to achieve, for example, greater stability in the gastrointestinal tract or to achieve a desired release rate. In addition, capsules containing powders, pills or granules can be further coated. Tablets may be scored to facilitate splitting of administration. Alternatively, dosage forms of the present invention may be unit dosage forms, wherein one unit dosage form is intended to deliver one therapeutic dose per administration, or wherein multiple unit dosage forms are intended to deliver a total therapeutic dose per administration.

The term "administration" refers to the manner in which the therapeutic compound is presented to an individual.

The term "oral administration" refers to any method of administration wherein the therapeutic compound can be administered by the oral route by swallowing, chewing or sucking the oral dosage form. Solid oral dosage forms are traditionally intended to substantially release and/or deliver the active drug in the gastrointestinal tract behind the oral and/or buccal cavity. Examples of solid dosage forms include conventional tablets, capsules, caplets, and the like.

The term "individual" or "patient" is intended to include animals. Examples of individuals include mammals such as humans, dogs, cattle, horses, pigs, sheep, goats, cats, mice, rabbits, rats, and transgenic non-human animals. In certain embodiments, the individual is a human, such as a human (including male and female subjects, and including neonates, infants, juveniles, adolescents, adults, and elderly subjects).

The terms "comprising" and "including" as used herein have an open and non-limiting meaning unless otherwise specified.

Unless otherwise indicated herein or clearly contradicted by context, the terms "a" and "the" and similar expressions are intended to encompass both the singular and the plural in the context of describing the invention (especially in the context of the scope of the following claims).

The terms "about" and "approximately" as used herein are used to provide flexibility of numerical range endpoints by providing that a given value may be "slightly above" or "slightly below" the endpoints to account for the visible differences in the measurements between different instruments, samples and sample preparations. The term "about" or "approximately" generally means within 10% of a given value or range, more preferably within 5%, and most preferably within 1%.

For convenience, various items, structural elements, constituent elements and/or materials as used herein may be presented in a list. However, these lists should be treated as if each member of the list is individually identified as a separate and unique member. Accordingly, individual members of a list should not be construed as factually equivalent merely on the basis that they are presented in a common group with any other member of the same list and no indication to the contrary is indicated. 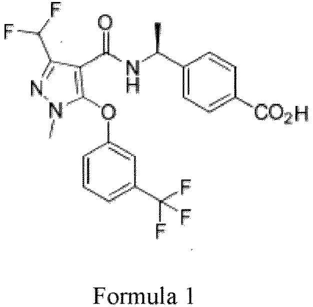 Compound: Compounds or pharmaceutically acceptable salts or esters, and their various forms of existence.

The compound of formula 1 4-{(1S)-1-[({3-(difluoromethyl)-1-methyl-5-[3-(trifluoromethyl)phenoxy]-4-1H-pyrazolyl} carbonyl)amino]ethyl}benzoic acid(AN0025) and its pharmaceutically acceptable salts are intended to be used in the pharmaceutical composition of the present invention. The compound, its pharmaceutically acceptable salts and its preparation are disclosed in WO2012039972A, the full text of which is incorporated in this disclosure by citation.

The compound of formula 1 may be present in the pharmaceutical compositions of the present invention in its free acid or pharmaceutically acceptable salt form. These salts can be prepared in situ during the final isolation and purification of the compound, or by reacting the base or acid functionality alone with a suitable organic or inorganic acid or base, respectively. Examples of acid addition salts that can be used to form pharmaceutically acceptable acids include inorganic acids such as hydrochloric acid, hydroboric acid, nitric acid, sulfuric acid and phosphoric acid and organic acids such as formic acid, acetic acid, trifluoroacetic acid, fumaric acid, tartaric acid, oxalic acid, maleic acid, methanesulfonic acid, succinic acid, malic acid, methanesulfonic acid, benzenesulfonic acid and p-toluenesulfonic acid, citric acid) and acidic amino acids (e.g. aspartic acid and glutamic acid). Pharmaceutically acceptable base salts include, but are not limited to, alkali and alkaline earth metal-based cations, such as sodium, lithium, potassium, calcium, magnesium, aluminum, etc.; and nontoxic ammonium, quaternary ammonium, and ammonia cations, including but not limited to ammonium, tetramethylammonium, tetraethylammonium, methylamine, dimethylamine, trimethylamine, triethylamine, ethylamine, etc. Other representative organic amines that can be used to form base addition salts include diethylamine, ethylenediamine, ethanolamine, diethanolamine, hexahydropyrazine, pyridine, picoline, triethanolamine, and the like; and basic amino acids such as arginine, lysine and ornithine.

According to the present invention, the compound of formula 1 or a pharmaceutically acceptable salt thereof may be present in the pharmaceutical composition ranging from about 20% to about 90%, about 30% to about 80%, about 35% to about 70% with respect to the total weight of the total composition. In one embodiment, the compound of formula 1 or a pharmaceutically acceptable salt thereof is present in a therapeutically effective amount ranging from about 40% to about 60% by weight of the total composition. Preferably, the compound of formula 1 or a pharmaceutically acceptable salt thereof is about 25%, about 30%, about 35%, about 40%, about 45%, about 50%, about 55%, about 60%, about 65%, about 70%, about 75%, about 80%, or about 85% by weight of the total composition.

In addition to the compound of formula 1 or a pharmaceutically acceptable salt thereof, the solid pharmaceutical composition of the present invention further comprises at least one pharmaceutically acceptable carrier for medicine. Examples of such carriers include, but are not limited to, fillers (or diluents), disintegrants, glidants, binders, stabilizers, colorants, flavor enhancers, preservatives, or combinations thereof. One or more of the aforementioned excipients can be selected by one of ordinary skill in the art by routine experimentation and without any undue burden for the specific desired properties of the solid pharmaceutical composition. The amount of each carrier used can vary within ranges commonly used in the art.

Fillers suitable for use in the present invention are compatible with the ingredients of the pharmaceutical composition, i.e., they do not substantially reduce the chemical stability, physical stability, or biological activity of the pharmaceutical composition. Examples of pharmaceutically acceptable fillers include, but are not limited to, cellulose, modified cellulose (e.g., sodium carboxymethyl cellulose, ethyl cellulose, hydroxymethyl cellulose, hydroxypropyl cellulose), cellulose acetate , microcrystalline cellulose, cellulose powder, calcium phosphate, calcium hydrogen phosphate, starch (e.g. corn starch, potato starch), dextrin, pregelatinized starch, sugars (e.g. mannitol, sorbitol, lactose, dextran, sucrose, dextrose, powdered sugar, compressible sugar, etc.), talc, or any combination thereof. In one embodiment of the present invention, the filler is preferably microcrystalline cellulose, mannitol and/or lactose. In one embodiment of the present invention, the filler may be present ranging from about 5% to about 60%, about 10% to about 50%, about 15% to about 45%, about 20% to about 40%, based on the total weight of the composition. In a preferred embodiment of the present invention, the filler may be present in an amount of about 5%, about 10%, about 15%, about 20%, about 22%, about 25%, about 27.5%, about 30% based on the total weight of the composition , about 33%, about 34%, about 35%, about 37%, about 40%, about 42%, about 44%, about 46%, about 48%, about 50%, about 55%, or about 60%.

Examples of pharmaceutically acceptable disintegrants include, but are not limited to, starch; cellulose; calcium carboxymethylcellulose; alginates; gums; sodium carboxymethyl starch; cross-linked polymers such as cross-linked polyvinylpyrrolidone or crospovidone; croscarmellose sodium or croscarmellose sodium; calcium carboxymethyl cellulose; low-substituted hydroxypropyl cellulose; soy polysaccharide; sodium starch glycolate; and guar glue or a combination thereof. In one embodiment of the present invention, the disintegrant is preferably croscarmellose sodium, low-substituted hydroxypropyl cellulose and/or crospovidone. In one embodiment of the invention, the disintegrant may be present in an amount from about 0% to about 30%, about 0.1% to about 25%, about 1% to about 25%, about 1% to about 22.5%, about 5% to about 20% based on the total weight of the composition. In a preferred embodiment of the present invention, the disintegrant may be present in an amount of about 0.5%, about 1%, about 2.5%, about 5%, about 7.5%, about 10%, about 12%, about 14%, about 16%, about 18%, about 20%, about 21%, about 22%, about 23%, about 24%, about 25%, about 27.5%, or about 30% based on the total weight of the composition.

Examples of pharmaceutically acceptable binders include, but are not limited to, starch; cellulose and derivatives thereof, such as microcrystalline cellulose, hydroxypropyl cellulose, hydroxyethyl cellulose, hypromellose; sucrose; dextrose; corn syrup; polysaccharide; povidone; copovidone neutralized gelatin or a combination thereof. In one embodiment of the present invention, the binder is preferably hydroxypropyl cellulose, hypromellose, povidone and/or copovidone. In one embodiment of the present invention, the binder may be present in an amount from about 0% to about 10%, about 0% to about 8%, about 2% to about 6%, about 3% to about 5%, based on the total weight of the composition. In a preferred embodiment of the present invention, the binder may be present in an amount of about 0.5%, about 1%, about 2%, about 3%, about 3.5%, about 4%, about 4.5%, about 5%, about 5.5%, about 6%, about 7%, about 8%, about 9%, or about 10% based on the total weight of the composition.

Examples of pharmaceutically acceptable lubricants include, but are not limited to, magnesium stearate, sodium stearyl fumarate, talc, polyethylene glycol, glyceryl behenate, and combinations thereof. In one embodiment of the present invention, the lubricant is magnesium stearate and/or sodium stearyl fumarate. In one embodiment of the present invention, the lubricant may be present in an amount of about 0.1% to about 10%, about 0.5% to about 8%, about 1% to about 6%, based on the total weight of the composition. In a preferred embodiment of the present invention, the lubricant is present in an amount of about 0.5%, about 1%, about 2%, about 2.5%, about 3%, about 4%, about 5%, about 6%, about 6%, about 7%, about 8%, about 9%, or about 10%, based on the total weight of the composition.

A pharmaceutically acceptable glidant refers to a pharmaceutical excipient whose primary function is to improve the flowability of a product by reducing intergranular friction. Glidants can be selected from silicon-containing materials such as fumed silica gel, pyrogenic silica gel, and hydrated sodium aluminosilicate and talc. Preferably, the pharmaceutical composition of the present invention includes a glidant, preferably colloidal silica. In one embodiment of the present invention, the glidant may be from about 0% to about 5%, from about 0% to about 3%, from about 0% to about 1%, based on the total weight of the composition. In a preferred embodiment of the present invention, no glidant is contained. In another preferred embodiment, the glidant is about 0.5%, about 1%, about 1.5%, about 2%, about 2.5%, about 3%, about 3.5%, about 4%, about 4.5%, about 5%, based on the total weight of the composition.

In one embodiment, the solid pharmaceutical composition of the present invention comprises at least one pharmaceutically acceptable carrier selected from the group consisting of a filler present in an amount from about 5% to about 60% by weight of the composition, a disintegrant present in an amount from about 0% to about 30% by weight of the composition, a binder present in an amount from about 0% to about 8% by weight of the composition, and a lubricant present in an amount from about 0.1% to about 10% by weight of the composition, a glidant present in an amount from about 0% to about 5% by weight of the composition, or a combination thereof. The glidant may be colloidal silica.

In one embodiment, the solid pharmaceutical composition of the present invention comprises at least one pharmaceutically acceptable carrier selected from the group consisting of a filler present in an amount from about 10% to about 50% by weight of the composition, a disintegrant present in an amount from about 0.1% to about 25% by weight of the composition, a binder present in an amount from about 2% to about 6% by weight of the composition, and a lubricant present in an amount from about 0.5% to about 8% by weight of the composition, a glidant present in an amount from about 0% to about 3% by weight of the composition, or a combination thereof. The glidant may be colloidal silica.

Preferably, the filler is selected from microcrystalline cellulose, calcium phosphate, calcium hydrogen phosphate, starch, dextrin, pregelatinized starch, mannitol, sorbitol, lactose, dextran, sucrose, dextrose or combinations thereof; the binder is selected from cellulose ethers, povidone, copovidone, starch, corn syrup or combinations thereof, wherein the cellulose ethers include but are not limited to hypromellose, hydroxypropyl cellulose, methyl cellulose, hydroxyethyl cellulose; the disintegrant is selected from croscarmellose sodium, crospovidone, sodium carboxymethyl starch, calcium carboxymethyl cellulose, low-substituted hydroxypropyl cellulose or combinations thereof; the lubricant is selected from magnesium stearate, sodium stearyl fumarate, polyethylene glycol, glycerin, behenate, talc or combinations thereof. More preferably, the filler is selected from microcrystalline cellulose, lactose, mannitol, sorbitol or combinations thereof, and the binder is selected from hydroxypropyl cellulose, hypromellose, povidone, copovidone or combinations thereof; and the disintegrant is selected from croscarmellose sodium, crospovidone, sodium carboxymethyl starch, calcium carboxymethyl cellulose, low-substituted hydroxypropyl cellulose or combinations thereof; and the lubricant is selected from magnesium stearate, sodium stearyl fumarate, or combinations thereof. Preferably, the dissolution RSD of the solid pharmaceutical composition is less than 20% at any time point from 5 minutes to 60 minutes. More preferably, the solid pharmaceutical composition has a dissolution RSD of less than 10% at any time point from 5 minutes to 60 minutes.

In another embodiment, the solid pharmaceutical composition of the present invention comprises a granule made from the granulation part having an inner phase and an external phase made from the additional part adjacent to the granule. The constituents of the granules and the external phase and the content of each constituent may be the same or different. The proportions of the granules and the external phase in the total weight of the solid pharmaceutical composition can be adjusted by researchers according to the selected excipients and technological needs.

The present invention relates to a manufacturing process of a solid pharmaceutical composition.

In one embodiment, the pharmaceutical composition of the present invention is prepared by a dry granulation process. According to the present invention, a pharmaceutical composition can be prepared by a method comprising the steps of: grinding the granulation part comprising Compound 1 or a pharmaceutically acceptable salt thereof together with at least one pharmaceutically acceptable carrier, grinding and pressing solid material to form a plurality of granules with an internal phase, mixing the granules with the additional part, and optionally compressing the final mixture into tablets. Rolling uses a device that basically utilizes two rollers that roll towards each other. A compacting force is applied to the milled granules fed into the roller compactor by a screw conveyor system.

In the present invention, unless indicated to the contrary, the "granulation part" refers to the raw material part used to prepare the granules having an internal phase; the "additional part" refers to the excipient part that are used for mixing with the granules having an internal phase to prepare and obtain the the solid pharmaceutical preparation, which constitutes the outer phase of the solid pharmaceutical preparation adjacent to the granules having the inner phase.

Various methods of granulation, sieving and mixing are known in the art, such as free-fall mixing or tumble mixing, compression into tablets on a single punch or rotary tablet press, or compaction on roller compaction equipment.

The sieving or screening step can be accomplished using any suitable means, such as the use of oscillating sieving or manual/vibrating sieves or a commercially available screening mill equipped with a suitably sized screen. One of ordinary skill in the art will have the experience and knowledge of how to determine and select an appropriate size screen for the s sieving or screening step. For example, the sieving or screening step can be carried out using a screening mill equipped with an appropriately sized screen.

The rolling compaction step is accomplished using a roller compactor having a compaction force in the range of about 3.6 MPa to about 19.4 MPa, preferably about 4.0 MkPa to 10.0 MPa, and most preferably about 7.0 MPa. The device used is preferably a roller compactor. The screw speed of the equipment is properly adjusted to ensure the proper quality of the rolled material. The roll speed is adjusted appropriately to ensure the proper quality of the rolled material.

The milling/screening step can be accomplished using any suitable means. Typically, the milled material (which forms granules with an internal phase) is passed through a screening mill or oscillating screen/mill having a screen with a mesh size of at least 0.5mm, eg, 0.6mm, 0.8mm, 1.2mm, or 2mm and milled. This milling/screening step produces multiple granules.

The step of mixing the granules with the additional part can be accomplished in a hopper mixer. The tablet solid dosage form can then be obtained by being compressed using a suitable rotary press at the desired target tablet weight.

In a preferred embodiment, the present invention relates to a solid oral dosage form comprising a pharmaceutical composition of the present invention and coated with a coating. Suitable coatings are known in the art and are commercially available or can be manufactured according to known methods. Typically, the coating material is a hydrophilic polymer such as polyvinyl alcohol, hydroxypropyl cellulose, hydroxymethyl cellulose, and hypromellose, among others. The coating composition ingredients may include conventional amounts of plasticizers, such as polyethylene glycol, triethyl citrate, diethyl phthalate, propylene glycol, glycerin; and opacifiers, such as titanium dioxide; and/or colorants , such as iron oxide, aluminum lake, etc. Typically, the coating material is applied, for example, in an amount that provides a coating in the range of from about 1% to about 6% of the total solid oral dosage form. These products are individually prepared dry premixes of film-forming polymers, opacifiers, colorants and plasticizers, which are further processed into aqueous coating suspensions. Preferably, the coating is applied to achieve a weight gain of the solid oral dosage form of about 1% to 10% of the total composition and preferably of about 2% to 6% of the total composition. In one embodiment of the present invention, the coating material is Opadry^{®}, and the coating weight comprises about 3% to 4% of the total weight of the solid oral dosage form.

In another embodiment, the present invention relates to a process for the manufacture of a solid pharmaceutical composition, comprising the steps of:
(a) Compound 1 or a pharmaceutically acceptable salt thereof and at least one pharmaceutically acceptable carrier are mixed together and uniformly mixed,
(b) rolling the sieved or screened material to form a compacted material,
(c) grinding the compacted material to form a plurality of granules,
(d) mixing the granules with the additional part to form a final mixture, and
(e) optionally compressing the final mixture into tablets.

In one embodiment, the present invention may further comprise a process for the manufacture of a coated tablet, after compressing the final mixture into a tablet according to the steps described above, further comprising the step of optionally applying a coating to the tablet.

In another embodiment, the pharmaceutical composition of the present invention is prepared by a wet granulation process. According to the present invention, a pharmaceutical composition can be prepared by a method comprising the steps of: screening and weighing compound AN0025 and each pharmaceutically acceptable carrier; adding a granulation fluid with or without a binder while Compound AN0025 and a pharmaceutically acceptable carrier are mixed with a suitable mixing speed for an appropriate amount of time and the mixture is chopped into granules, wherein the granulation fluid may be a binder prepared by dispersing the binder in a solvent (water) solution, or simply a wetting agent, such as water; drying the granules; blending the granules with extragranular excipients for an appropriate amount of time; then compressing into tablet solid dosage forms at the desired target tablet weight using a suitable rotary press.

In another embodiment, the present invention relates to a process for the manufacture of a solid pharmaceutical composition, comprising the steps of:
(a) Compound 1 or a pharmaceutically acceptable salt thereof and at least one pharmaceutically acceptable carrier are mixed together and mixed evenly,
(b) While adding the granulation fluid, compound AN0025 and a pharmaceutically acceptable carrier are mixed for an appropriate amount of time at a suitable mixing speed and the mixture is chopped into granules,
(c) drying the granules,
(d) sieving to obtain dry granules of target particle size,
(e) mixing the granules with the additional part to form the final mixture, and
(f) optionally compressing the final mixture into tablets.

In one embodiment, the particle size of the compound of formula 1 in the solid pharmaceutical composition tablet of the present invention is D90 of less than about 200 microns; in one embodiment, the particle size of the compound of formula 1 in the solid pharmaceutical composition tablet of the present invention is D90 of less than about 100 microns; in a preferred embodiment, the compound of formula 1 in the solid pharmaceutical composition tablet of the present invention has a particle size of a D90 of from about 10 microns to about 80 microns.

In one embodiment, the present invention may further comprise a process for the manufacture of coated tablets, after compressing the final mixture into tablets according to the steps described above, further comprising the step of optionally applying a coating to the tablets.

In one embodiment, the solid pharmaceutical composition tablet of the present invention contains about 50 mg to about 500 mg of the compound of formula 1; in another embodiment, the solid pharmaceutical composition tablet of the present invention contains about 100 mg to about 300 mg of the compound of formula 1. Preferably, the solid pharmaceutical composition tablet of the present invention contains about 125 mg to about 250 mg of the compound of formula 1.

The tablet solid pharmaceutical composition of the present invention obtained by dry granulation or wet granulation shows the following advantages:
(1) The preparation of the pharmaceutical composition has a rapid dissolution rate;
(2) The formulation of the pharmaceutical composition has a low dissolution RSD;
(3) The preparation of the pharmaceutical composition is suitable for APIs of different particle sizes;
(4) The in vivo exposure of the preparation of the pharmaceutical composition has a low individual difference.

The pharmaceutical composition of the present invention and its solid dosage form can be used to treat cancer, especially multiple sclerosis, rheumatoid arthritis, or cancer that can be beneficially treated by inhibiting EP4. Examples of cancers suitable for treatment with the solid pharmaceutical compositions of the present invention include, but are not limited to, for example: skin cancer, breast cancer, colorectal cancer, prostate cancer, kidney cancer, ovarian cancer, cervical cancer, endometrial cancer, glioblastoma, lung cancer, head and neck cancer, medulloblastoma, and urethral cancer. Other diseases that can be treated with the solid pharmaceutical compositions of the present invention are disclosed in WO2012039972A, which is incorporated herein by reference in its entirety.

In one embodiment, the present invention provides a solid pharmaceutical composition of the present invention for use in the treatment of cancer.

In one embodiment, the present invention provides the use of a solid pharmaceutical composition of the present invention in the manufacture of a medicament for the treatment of cancer.

In one embodiment, the present invention provides a method of treating cancer comprising administering to a patient suffering from the cancer a solid composition of the present invention comprising a therapeutically effective amount of a compound of formula (I) or a pharmaceutically acceptable salt thereof.

The details of one or more embodiments of the invention are set forth in the accompanying description above. Although methods and materials similar or equivalent to those described herein can be used in the practice or testing of the present invention, the preferred methods and materials are now described. Other features, objects and advantages of the present invention will be apparent from the description and claims. In the specification and the appended claims, the singular includes plural referents unless the context clearly dictates otherwise. Unless otherwise defined, all technical and scientific terms used herein have the same meaning as commonly understood by one of ordinary skill in the art to which this invention belongs. All patents and publications cited in this specification are incorporated by reference. The following examples are presented to more fully illustrate preferred embodiments of the present invention. These examples should in no way be construed to limit the scope of the disclosed patent as defined by the appended claims.

### EXAMPLES

### 1. Dissolution testing method:

### Dissolution parameters

Device: United States Pharmacopoeia and Chinese Pharmacopoeia Device II (Paddle)
Speed: 50 rpm
Dissolution Medium: pH 6.8 Phosphate Buffered Solution
Dissolution volume: 900 mL
Temperature: 37 ± 0.5 °C

### HPLC Detection conditions

Detection wavelength: 234 nm
Chromatographic column: YMC-Triart Cis 75×4.6 mm, 3 µm
Column temperature: 40 °C
Solvent A: Water / 70% perchloric acid (1000/1, v / v)
Solvent B: Acetonitrile / 70% perchloric acid (1000/1, v / v)
Elution gradient:

| Time (min) | A (%) | B (%) |
|---|---|---|
| 0.0 | 50 | 50 |
| 3.5 | 50 | 50 |
| Flow rate: 1.5 mL / min | | |
| Injection volume: 5 µL | | |

### Pharmacokinetic parameters test method

In the present invention, the pharmacokinetic parameters of the comparative examples and examples were measured by chromatography and mass spectrometry, and the details of the test conditions are listed in Table 1a and Table 1b.

**Table 1a. Chromatographic conditions**

| | | | | | |
|---|---|---|---|---|---|
| | Chromatograph | ACE Excel2 Super C18 (50×2.1 mm) | | | |
| Injection volume: | | 1 µL | | | |
| | Flow rate: | 0.500 mL·min⁻¹ | | | |
| | Mobile phase: | Mobile phase A | Water (0.1% formic acid) | | |
| | Mobile phase B | | Acetonitrile (0.1% formic acid) | | |
| Needle wash: | Methanol (100%) | | | | |
| Elution procedure: | Initial mobile phase (%B) ratio: 40 | | | | |
| | 40 | | | | |

| | Time (min) | Module | | Event | Parameter |
|---|---|---|---|---|---|
| | 0.50 | Pump | | %B | 40 |
| | 1.50 | Pump | | %B | 98 |
| | 2.40 | Pump | | %B | 98 |
| | 2.41 | Pump | | %B | 40 |
| | 3.50 | System Controller | | Stop | |
| Retention time: | AN0025 | ∼1.49 min | | Internal standard AN00251001 | ∼1.48 min |

| | | | | | |
|---|---|---|---|---|---|
| Note: AN00251001 is the deuterated compound of AN0025. | | | | | |

**Table 1b. mass spectrometry conditions**

| | | | | | | | |
|---|---|---|---|---|---|---|---|
| Scan Type: | | | MRM | | | | |
| Ionization Model: | | | Positive | | | | |
| Ion Source: | | | ESI | | | | |
| Collision Gas Pressure (CAD): | | | 12 psi | | | | |
| Curtain Gas Pressure (Curtain Gas): | | | 20 psi | | | | |
| Atomizing gas (GS1): | | | 55 psi | | | | |
| Auxiliary gas (GS2): | | | 55 psi | | | | |
| Ionspray Voltage: | | | 5500 V | | | | |
| Atomizing gas Temperature (Temperature): | | | 550 °C | | | | |

| Compound name | Q1 | Q3 | Dwell Time (msec) | DP (V) | EP (V) | CE (eV) | CXP (V) |
|---|---|---|---|---|---|---|---|
| AN0025 | 484.3 | 316.1 | 100 | 110 | 10 | 30 | 15 |
| AN00251001(In ternal standard) | 490.3 | 322.1 | 100 | 100 | 10 | 29 | 15 |

### 2. Comparative Example 1

Capsules of Comparative Example 1 were prepared according to the ingredients and contents as described in Table 2a.

**Table 2a. Prescription component of Comparative Example 1**

| Ingredient name | Theoretical prescription volume (mg/capsule) | | | weight percentage %W/W |
|---|---|---|---|---|
| Contents (granules) | | | | |
| AN0025 APIs | 75.0 | 125.0 | 200.0 | 50 |
| mannitol | 30.0 | 50.0 | 80.0 | 20 |
| low-substituted hydroxypropyl cellulose | 24.0 | 40.0 | 64.0 | 16 |
| hydroxypropyl cellulose | 4.5 | 7.5 | 12.0 | 3 |
| carboxymethylcellulose calcium | 7.5 | 12.5 | 20.0 | 5 |
| microcrystalline cellulose | 7.5 | 12.5 | 20.0 | 5 |
| magnesium stearate | 1.5 | 2.5 | 4.0 | 1 |
| total weight of capsule contents | 150.0 | 250.0 | 400.0 | - |

| Capsule | | | | |
|---|---|---|---|---|
| hypromellose capsule shell | 2# | 0# | 0# | - |

For the AN0025 capsule of Comparative Example 1, its manufacturing process consists of six steps: mixing, lubricating, granulating, sizing, capsule filling and weight sorting. AN0025 APIs, mannitol, low-substituted hydroxypropyl cellulose, hydroxypropyl cellulose, calcium carboxymethyl cellulose and microcrystalline cellulose were mixed into a high shear mixer or hopper mixer and mixed evenly. Magnesium stearate was then added to the above mixture for lubrication. Use a roller compactor to compress the mixture into long strips. Long strips were sieved to granulate (the mesh aperture is 1.5 mm). Use a capsule filling machine to fill the whole granules into hypromellose (HPMC) capsules, and use a checkweigher to remove overweight and underfilled capsules.

**Table 2b. Mean dissolution profile of 125mg capsules (Comparative Example 1) during stability study**

| Stability conditions | Dissolution time point/min | | | | | | | |
|---|---|---|---|---|---|---|---|---|
| | 5 | 10 | 15 | 20 | 30 | 45 | 60 | 75 |
| Initial | 0.0 | 1.6 | 21.2 | 44.0 | 86.8 | 94.2 | 96.0 | 99.6 |
| 25°C/60%RH, 1 month | 0.0 | 0.1 | 23.2 | 54.3 | 81.6 | 93.3 | 96.4 | ND |
| 25°C/60%RH, 3 months | 0.3 | 3.3 | 23.7 | 56.2 | 87.3 | 94.9 | 95.6 | 96.4 |
| 25°C/60%RH, 6 months | 0.0 | 0.6 | 29.4 | 54.1 | 77.9 | 91.2 | 94.3 | 97.9 |
| 25°C/60%RH, 12 months | 0.0 | 1.4 | 25.6 | 63.0 | 86.1 | 96.0 | 97.1 | 97.7 |
| 25°C/60%RH, 18 months | 0.0 | 0.4 | 33.6 | 54.3 | 81.1 | 88.3 | 91.6 | 95.2 |
| 25°C/60%RH, 24 months | 0.0 | 0.0 | 21.2 | 55.7 | 83.1 | 91.7 | 94.2 | 95.2 |
| 25°C/60%RH, 30 months | 0.0 | 0.3 | 19.9 | 44.5 | 68.0 | 81.8 | 88.1 | 94.2 |
| 25°C/60%RH, 36 months | 0.0 | 2.1 | 25.5 | 55.1 | 83.2 | 94.5 | 96.8 | 97.7 |
| 40°C/75%RH, 1 months | 0.0 | 4.5 | 16.0 | 37.6 | 83.8 | 96.0 | 98.4 | 100.6 |
| 40°C/75%RH, 3 months | 0.0 | 5.3 | 36.3 | 64.2 | 85.1 | 92.5 | 95.3 | 96.8 |
| 40°C/75%RH, 6 months | 0.0 | 0.6 | 14.2 | 37.7 | 73.5 | 93.9 | 96.2 | 98.9 |

| | | | | | | | | |
|---|---|---|---|---|---|---|---|---|
| ND: Due to experimental error, not determined | | | | | | | | |

Table 2b shows the mean value of the dissolution profiles of the 125 mg capsules of Comparative Example 1 during the stability study. Under different storage conditions (long-term stability condition 25°C/60% relative humidity, accelerated stability condition 40°C/75% relative humidity), the results measured at different time points from 1 to 36 months are quite different. This difference is not a trend, but fluctuates up and down, indicating that even the average dissolution value cannot reflect the real dissolution state of the drug.
Among them, the dissolution profile determination results of another batch of Comparative Example 1 are shown in Table 2c, where SD is the standard deviation, and RSD is the relative standard deviation. Unless otherwise stated, SD and RSD described herein have the same definitions and calculation methods as those in Table 2c. Figure 2c shows that the detection results of different capsule samples at the same time point are quite different, and the RSD is larger. The general acceptance standard for technicians in the industry to determine the dissolution profile is that the RSD of the dissolution test value at the first time point is not more than 20%, and the RSD of the dissolution test value at the other time points is not more than 10%.

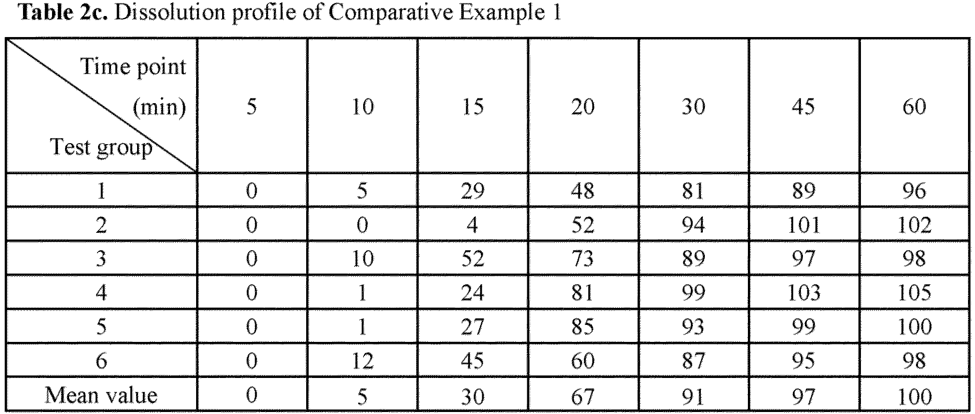

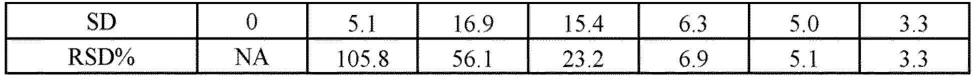

The above results show that the dissolution RSD of the capsules is relatively large, and the detection results of different batches or the same batch at different times have large fluctuations.

### 3. Comparative Example 2

Capsules of Comparative Example 2 were prepared according to the ingredients and contents as described in Table 3. Comparative Example 2 used the same components and contents as Comparative Example 1, and was filled into different hollow capsules.

**Table 3a. Prescription component of Comparative Example 2**

| Ingredient name | Theoretical prescription volume (mg/capsule) | weight percentage %W/W |
|---|---|---|
| Contents (granules) | | |
| AN0025 APIs | 125.0 | 50 |
| mannitol | 50.0 | 20 |
| low-substituted hydroxypropyl | 40.0 | 16 |
| hydroxypropyl cellulose | 7.5 | 3 |
| carboxymethylcellulose calcium | 12.5 | 5 |
| microcrystalline cellulose | 12.5 | 5 |
| magnesium stearate | 2.5 | 1 |
| total weight of capsule contents | 250.0 | - |

| Capsules | | |
|---|---|---|
| gelatin hollow Capsules | 0# | - |

The AN0025 capsule of Comparative Example 2, its manufacturing process consists of six steps: mixing, lubricating, granulating, sizing, capsule filling and weight sorting. Combine AN0025 API, mannitol, low-substituted hydroxypropyl cellulose, hydroxypropyl cellulose, carboxymethyl cellulose calcium, and microcrystalline cellulose into a high shear mixer. The magnesium stearate is mixed with the mixture using a high shear mixer. Use a roller compactor to compress the mixture into long strips. Long strips are sieved to granulate (the mesh aperture is 1.5 mm). Use a capsule filling machine to fill the whole granules into gelatin hollow capsules, and use a checkweigher to remove overweight and underfilled capsules.

**Table 3b. Dissolution behavior in comparative example 2 stability study**

| Stability | Dissolution | 5min | 10 min | 15 min | 20 min | 30 min | 45 min | 60 min |
|---|---|---|---|---|---|---|---|---|
| Initial | Mean value/% | 42 | 82 | 87 | 88 | 91 | 94 | 95 |
| | RSD/% | 32.6 | 5.1 | 2.8 | 2.5 | 2.6 | 2.0 | 1.7 |
| 40°C/75%RH 30 days | Mean value/% | 19 | 41 | 50 | 54 | 58 | 61 | 64 |
| | RSD/% | 72.7 | 44.0 | 32.3 | 27.6 | 24.9 | 22.8 | 22.1 |

Table 3b shows that, compared with Comparative Example 1 using HPMC capsules, Comparative Example 2 using gelatin capsules can dissolve faster, and its dissolution RSD is also greatly improved, but the RSD in 5min is still more than 20%. Moreover, during the accelerated stability study at 40°C/75% relative humidity, the capsule of Comparative Example 2 shows a relatively obvious decrease in dissolution.

### 4. Comparative Example 3

In order to improve the higher RSD of Comparative Example 1, the inventors prepared the capsules of Comparative Example 3 according to the ingredients and contents as described in Table 4a using different preparation process.

**Table 4a. Prescription component of Comparative Example 3**

| Ingredient name | weight percentage %W/W | Theoretical prescription volume(mg) |
|---|---|---|
| Granulation part | | |
| Compound AN0025 | 50 | 125 |
| lactose monohydrate | 27 | 67.5 |
| hydroxypropyl cellulose | 3 | 7.5 |
| microcrystalline cellulose | 7 | 17.5 |

| Additional part | | |
|---|---|---|
| low-substituted hydroxypropyl cellulose | 12 | 30 |
| magnesium stearate | 1 | 2.5 |

The preparation process is as follows:

### Step 1 Mixing

The weighed Compound AN0025, lactose monohydrate, hydroxypropyl cellulose and microcrystalline cellulose were added to high shear granulator and mixed for 2 minutes at 300 rpm.

### Step 2 Granulating

The parameters of the high shear granulator were set as the stirring speed of 200 r/min and the shearing speed of 800 r/min, and the soft material was prepared by slowly adding purified water as a wetting agent. After all the wetting agents were added, continue to run at the same stirring speed and shearing speed for 2 to 3 minutes, and then discharge. The soft material was passed through a screen with an aperture of 2.0 mm to obtain wet granules.

### Step 3 Drying

The wet granules obtained in the above steps were dried to remove moisture.

### Step 4 Sizing

The granules obtained after drying were again sieved and sized to obtain granules with an internal phase.

### Step 5 Mixing II

The granules prepared above are mixed with low-substituted hydroxypropyl cellulose uniformly.

### Step 6 Lubricating II

The mixture prepared above was then mixed with magnesium stearate uniformly.

### Step 7 Capsule filling

Fill the lubricated material into hypromellose (HPMC) capsules according to the target weight.

However, as shown in Table 4b, the capsules prepared by the wet granulation process still have high RSD.

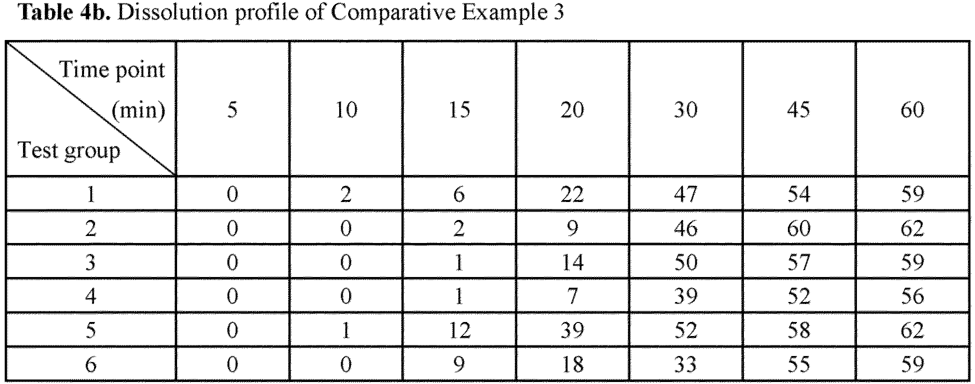

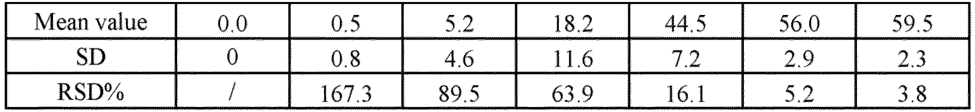

In order to fundamentally solve the problem of high RSD of the compound AN0025 formulation, the inventors implemented the following specific examples.

### 5. Example 1

The tablet of Example 1 of the present invention was prepared according to the ingredients and contents as described in Table 5a.

**Table 5a. Prescription component of Example 1**

| Ingredient name | weight percentage %W/W | Theoretical prescription volume(mg) |
|---|---|---|
| Granulation part | | |
| Compound AN0025 | 50 | 125 |
| mannitol | 20 | 50 |
| low-substituted hydroxypropyl cellulose | 16 | 40 |
| hydroxypropyl cellulose | 3 | 7.5 |
| carboxymethylcellulose calcium | 5 | 12.5 |
| microcrystalline cellulose | 5 | 12.5 |
| magnesium stearate | 0.5 | 1.25 |

| Additional part | | |
|---|---|---|
| magnesium stearate | 0.5 | 1.25 |

The preparation process is as follows:

### Step 1 Mixing

The weighed Compound AN0025, mannitol, low-substituted hydroxypropyl cellulose, hydroxypropyl cellulose, carboxymethylcellulose calcium and microcrystalline cellulose mixed evenly_{∘}

### Step 2 Lubricating I

Magnesium stearate from the granulation part are added to the mixture in step 1 and mixed evenly_{∘}

### Step 3 Granulating

Use a roller compactor to press the mixture into long strips, and the rolling parameters are: compaction force 10Mpa; roll speed: 4rpm; gap width 1.5mm.

### Step 4 Sizing

The long strips are sieved and granulated through a coarse screen (pore size 2.0 mm) and a fine screen (pore size 0.6 mm), respectively, to obtain granules with an internal phase.

### Step 5 Lubricating II

The granules prepared above were mixed with an additional part of magnesium stearate.

### Step 6 Tabletting

At the desired target tablet weight of 250 mg, a tablet machine is used to compress into tablets of appropriate shape.

The prescription components of Example 1 are the same as those of Comparative Example 1 and Comparative Example 2, except that they are prepared in different dosage forms. The dissolution data are shown in Table 5b, and the dissolution RSD at all time points is not more than 10%, which is significantly improved compared to Comparative Example 1 and Comparative Example 2. In addition, FIG. 2 also shows that the drug dissolution rate of the tablet of Example 1 is higher than that of the capsule of Comparative Example 1.

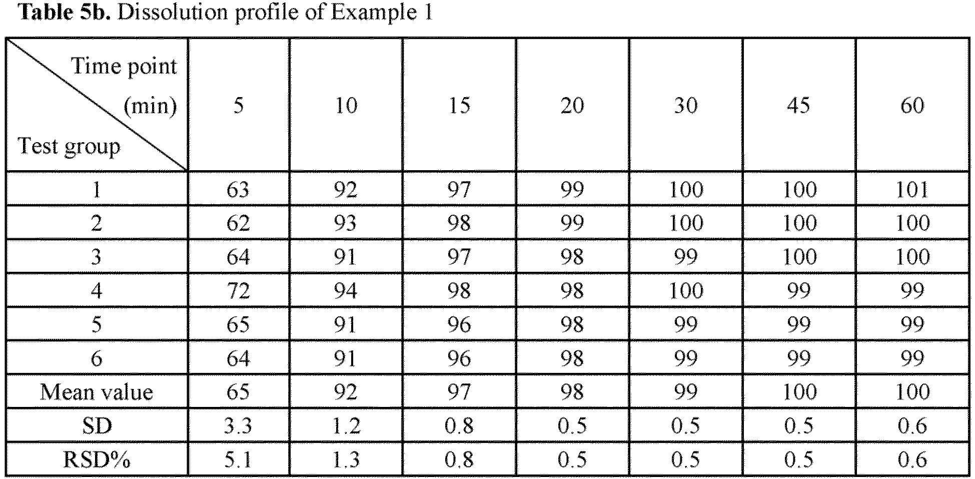

### 6. Example 2

The tablet of Example 2 of the present invention was prepared according to the ingredients and contents as described in Table 6a.

**Table 6a. Prescription component of Example 2**

| Ingredient name | weight percentage %W/W | Theoretical prescription volume(mg) | |
|---|---|---|---|
| Granulation part | | | |
| Compound AN0025 | 50 | 125 | 250 |
| lactose monohydrate | 27 | 67.5 | 135 |
| hydroxypropyl cellulose | 3 | 7.5 | 15 |
| microcrystalline cellulose | 7 | 17.5 | 35 |

| Additional part | | | |
|---|---|---|---|
| low-substituted hydroxypropyl cellulose | 12 | 30 | 60 |
| magnesium stearate | 1 | 2.5 | 5 |

The preparation process is as follows:

### Step 1 Mixing

The weighed Compound AN0025, lactose monohydrate, hydroxypropyl cellulose and microcrystalline cellulose were added to high shear granulator and mixed for 2 minutes at 300 rpm.

### Step 2 Granulating

The parameters of the high shear granulator were set as the stirring speed of 200 rpm and the shear speed of 800 rpm, and the soft material was prepared by slowly adding purified water as a wetting agent. After all the wetting agents were added, continue to run the same stirring speed and shearing speed for 2 to 3 minutes, and then discharge. The soft material was passed through a screen with an aperture of 2.0 mm to obtain wet granules.

### Step 3 Drying

The wet granules obtained in the above steps were dried to remove moisture.

### Step 4 Sizing

The granules obtained after drying were sieved to granulate again to obtain granules with an internal phase.

### Step 5 Mixing II

Mix the granules prepared above with low-substituted hydroxypropyl cellulose uniformly.

### Step 6 Lubricating II

The mixture prepared above was then mixed with magnesium stearate uniformly.

### Step 7 Tableting

Use a tablet press to compress tablets into the appropriate shape based on the target tablet weight.

The dissolution data are shown in Table 6b, and the dissolution RSD of all time points does not exceed 10%.

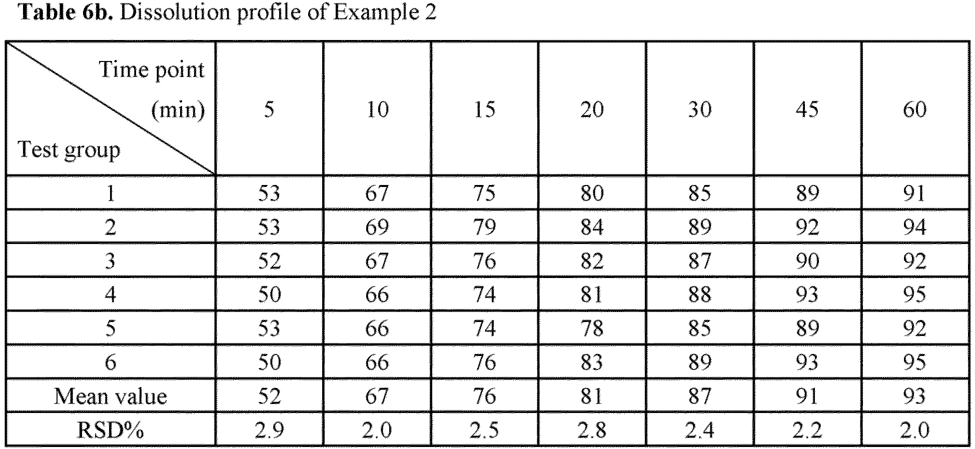

**Table 6c. Dissolution behavior in stability studies of Example 2**

| Stability | Dissolution | 5min | 10 min | 15 min | 20 min | 30 min | 45 min | 60 min |
|---|---|---|---|---|---|---|---|---|
| Initial | Mean value/% | 52 | 67 | 76 | 81 | 87 | 91 | 93 |
| | RSD/% | 2.9 | 2.0 | 2.5 | 2.8 | 2.4 | 2.2 | 2.0 |
| 40°C/75%RH, 30 days | Mean value/% | 49 | 63 | 71 | 76 | 82 | 86 | 89 |
| | RSD/% | 4.1 | 5.4 | 4.9 | 5.0 | 4.3 | 3.8 | 3.5 |

The prescription components of Example 2 and Comparative Example 3 are the same as the preparation process, and the difference is that they are prepared into different dosage forms. The dissolution profile comparison example of Example 2 is shown in Figure 2 of the description. Similar to the experimental results of Example 1, the drug dissolution rate of the tablet of Example 2 is also higher than that of the capsule of Comparative Example 3.

Example 2 was relatively stable during the stability study process, with no significant decrease, as shown in Table 6c. In contrast, the dissolution of Comparative Example 2 decreased significantly during the stability study, as shown in Table 3b. This result demonstrates the advantages of the tablet from a stability standpoint.

Examples 1 and 2 show that no matter whether it is dry granulation or wet granulation, the tablet of the present invention can obviously improve the problem of high RSD of capsules, and this improvement has nothing to do with the preparation process.

### 7. Example 3

By changing the ratio of each ingredient in the prescription, the tablet of Example 3 of the present invention was prepared according to the ingredients and contents as described in Table 7a.

**Table 7a. Prescription component of Example 3**

| Ingredient name | weight percentage %W/W | Theoretical prescription volume(mg) |
|---|---|---|
| Granulation part | | |
| Compound AN0025 | 50 | 250 |
| mannitol | 10 | 50 |
| hydroxypropyl cellulose | 4 | 20 |
| carboxymethylcellulose calcium | 1 | 5 |
| microcrystalline cellulose | 30 | 150 |
| sodium stearyl fumarate | 1 | 5 |

| Additional part | | |
|---|---|---|
| sodium stearyl fumarate | 4 | 20 |

| Coating | | |
|---|---|---|
| Opadry^{®} | coating weight gain 4% | 20 |

The preparation process is as follows:

### Step 1 Mixing

The weighed Compound AN0025, mannitol, hydroxypropyl cellulose, carboxymethylcellulose calcium and microcrystalline cellulose were added to hopper mixer and mixed evenly.

### Step 2 Lubricating I

Use a hopper mixer to mix the magnesium stearate in the granulation part with the mixture in step 1 uniformly.

### Step 3 Granulating

The mixture was pressed into long strips using a roller compactor, and the roller compaction parameters were: compaction force: 7Mpa; roll speed: 2rpm; gap width: 1.5mm.

### Step 4 Sizing

The long strips were crushed and sieved through a 0.8 mm screen to granulate to obtain granules with an internal phase.

### Step 5 Lubricating II

The granules prepared above were mixed with an additional part of magnesium stearate.

### Step 6 Tabletting

At the desired target tablet weight of 500 mg, use a tablet press to compress into tablets of the appropriate shape.

### Step 7 Coating

Fully disperse Opadry in purified water, configure a coating solution, and perform film coating through a coating machine, with a target coating weight gain of 4%.

The dissolution data are shown in Table 7b. Except that the dissolution RSD at the first time point was slightly higher (but not more than 20%), the dissolution RSD at the other points did not exceed 10%.

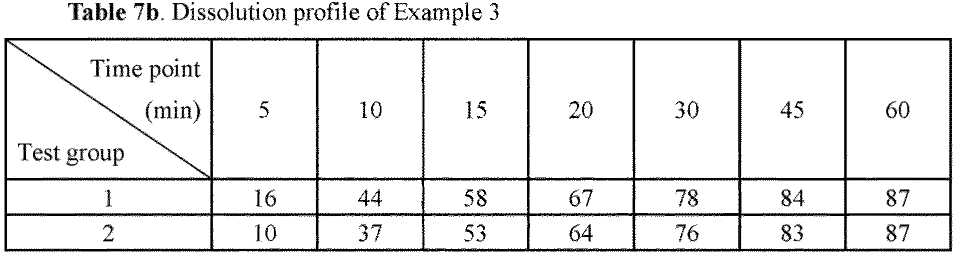

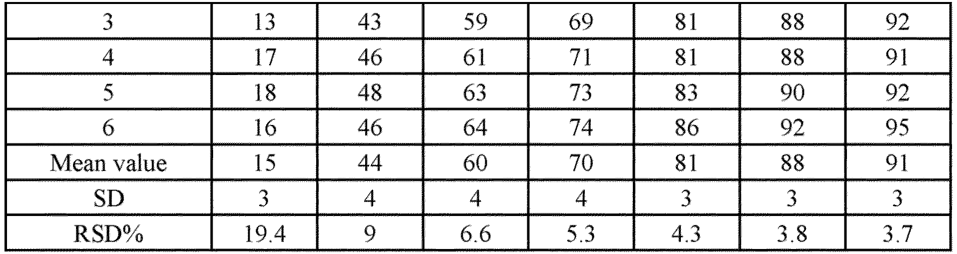

### 8. Example 4

Coated tablets of Example 4 were prepared according to the ingredients and amounts as described in Table 8a.

**Table 8a. Prescription component of Example 4**

| Ingredient name | weight percentage %W/W | Theoretical prescription volume (mg) |
|---|---|---|
| Granulation part | | |
| Compound AN0025 | 50 | 250 |
| mannitol | 8.88 | 44.4 |
| hydroxypropyl cellulose | 4 | 20 |
| Sodium croscarmellose | 5 | 25 |
| microcrystalline cellulose | 26.62 | 133.1 |
| sodium stearyl fumarate | 1 | 5 |

| Additional part | | |
|---|---|---|
| sodium stearyl fumarate | 4 | 20 |
| colloidal silica | 0.5 | 2.5 |

| Coating | | |
|---|---|---|
| Opadry^{®} | coating weight gain 4% | 20 |

The preparation process is as follows:

### Step 1 Mixing

The weighed Compound AN0025, mannitol, hydroxypropyl cellulose, sodium croscarmellose and microcrystalline cellulose were added to hopper mixer and mixed evenly.

### Step 2 Lubricating I

Use a hopper mixer to mix the magnesium stearate in the granulation part with the mixture in step 1 until uniform.

### Step 3 Granulating

Use a roller compactor to press the mixture into long strips, and the rolling parameters are: compaction force 7Mpa; roll speed: 2rpm; gap width 1.5mm.

### Step 4 Sizing

The long strips are crushed and sieved through a 0.8 mm screen to obtain granules with an internal phase.

### Step 5 Lubricating II

The granules prepared above were mixed with an additional part of magnesium stearate.

### Step 6 Tabletting

At the desired target tablet weight of 500 mg, use a tablet press to compress into tablets of the appropriate shape.

### Step 7 Coating

Fully disperse Opadry in purified water, prepare a coating solution, and perform film coating through a coating machine with a target coating weight gain of 4%.

The dissolution data are shown in Table 8b, and the dissolution RSD of all Time points does not exceed 10%.

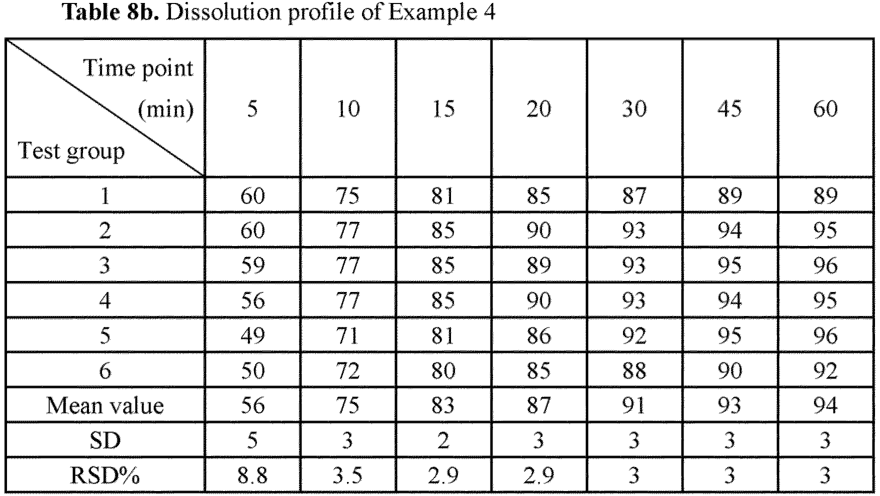

After being placed at 40°C/75% relative humidity for 1 month, the dissolution rate did not change significantly, as shown in Table 8c.

**Table 8c. Dissolution behavior in stability studies of Example 4**

| | | | | | | | |
|---|---|---|---|---|---|---|---|
| Time point/min | 5 | 10 | 15 | 20 | 30 | 45 | 60 |
| initial | 56 | 75 | 83 | 87 | 91 | 93 | 94 |
| 40°C/75%RH, 1 month | 50 | 71 | 82 | 88 | 94 | 97 | 98 |

Tablets of Comparative Example 1, Example 3 and Example 4 were used for animal testing.

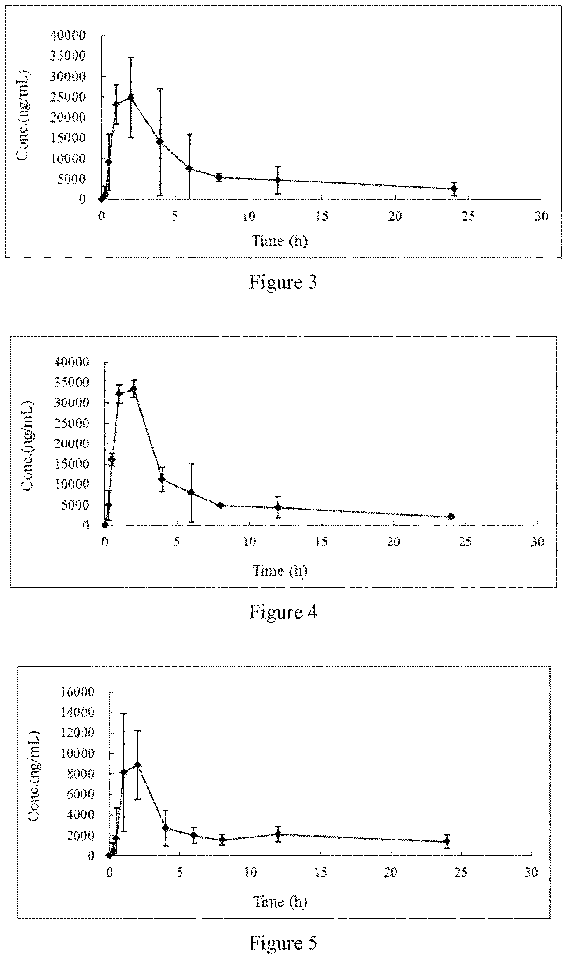

Table 8d shows the comparison of pharmacokinetic parameters of different preparations in beagle dogs when the dose of 750mg/dog is given, where AUC₀₋ₜ represents the area under the drug concentration-time curve from 0 to the last selected time point, AUC_{0-t_dose} represents the ratio of the area under the drug concentration-time curve at the time from 0 to the last selected time point to the dose. Unless otherwise stated, AUC₀₋ₜ and AUC_{0-t_dose} described herein have the same definitions and calculation methods as those in Table 8d. Compared with Comparative Example 1, the in vivo exposure (AUC) of the tablets of Example 3 and Example 4 was comparable, but the inter-individual difference (SD) was smaller, which was more consistent with the in vitro dissolution data, suggesting that the tablets may have better efficacy and safety clinically. Comparative Example 1, Example 4, the drug concentration-time curves in beagle dogs at a dose of 750 mg/dog are shown in Figures 3 and 4, respectively.

**Table 8d. Comparison of pharmacokinetic parameters of different preparations in beagle dogs at a dose of 750 mg**

| Formulation prescription | | Comparative Example 1 | | Example 3 | | Example 4 | |
|---|---|---|---|---|---|---|---|
| Dosage | | **750 mg/dog** | | | | | |

| Pharmacokinetic parametersPharmacokinetic parameters | Unit | Mean | SD | Mean | SD | Mean | SD |
|---|---|---|---|---|---|---|---|
| Cₘₐₓ | ng/mL | 29400 | 5860 | 29800 | 12800 | 33400 | 2090 |
| AUC₀₋ₜ | ng*h/mL | 171000 | 74900 | 150000 | 6030 | 181000 | 41000 |
| AUC_{0-t_dose} | h*kg*ng/mL/mg | 1840 | 760 | 1700 | 72.8 | 1990 | 428 |

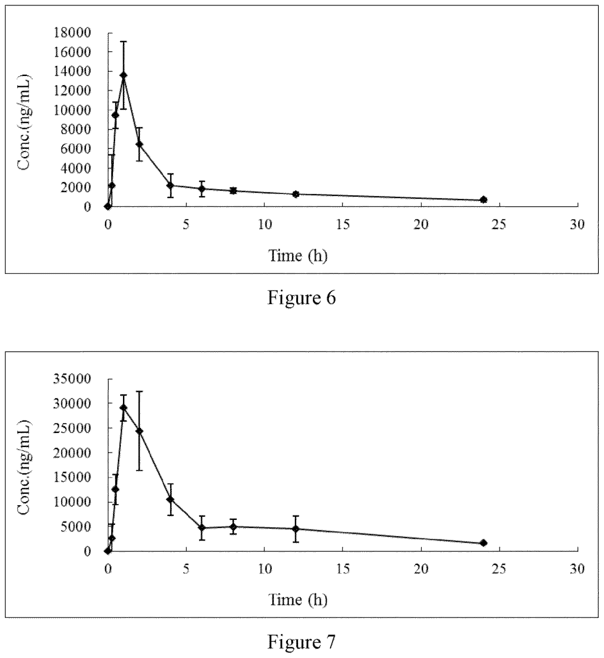

Table 8e shows the comparison of pharmacokinetic parameters of different preparations in beagle dogs when the administration dose is 250 mg/dog. Compared with the capsule of Comparative Example 1, the in vivo exposure (AUC) of the tablet of Example 4 was comparable, but the inter-individual difference (SD) was smaller, which was more consistent with the in vitro dissolution data, suggesting that the tablet has better efficacy and safety clinically. Comparative Example 1, Example 4 are shown in Figure 5 and Figure 6 respectively for the drug concentration-time curves in beagle dogs at a dose of 250 mg/dog.

**Table 8e. Comparison of pharmacokinetic parameters of different preparations in beagle dogs at a dose of 250 mg**

| Formulation prescription | | Comparative Example 1 | | Example 4 | |
|---|---|---|---|---|---|
| DosageDosage | | **250 mg/dog** | | | |

| Pharmacokinetic parameters | Unit | Mean | SD | Mean | SD |
|---|---|---|---|---|---|
| Cₘₐₓ | ng/mL | 11300 | 473 | 13600 | 3500 |
| AUC₀₋ₜ | ng*h/mL | 59300 | 16000 | 51400 | 8720 |
| AUC_{0-t_ dose} | h*kg*ng/mL/mg | 2030 | 527 | 1790 | 296 |

The above results show that the tablet of the present invention has similar in vivo exposure as the capsule, but the individual difference is significantly reduced, the RSD is reduced, and this improvement is not affected by the administered dose.

### 9. Example 4a

The prescription components and preparation process of Example 4a are the same as those of Example 4, but APIs with different particle sizes are used. The particle size difference of the APIs used in Example 4 and Example 4a is shown in Table 9a, wherein, D50 represents the particle size corresponding to the cumulative particle size distribution percentage of a sample reaching 50%, that is, granules with a particle size smaller than it account for 50%; D90 represents the particle size corresponding to the cumulative particle size distribution percentage of a sample reaching 90%, that is, granules with a particle size smaller than it account for 90%. Unless otherwise stated, D50 and D90 described herein have the same definitions as those in this embodiment.

**Table 9a. API particle size information of Example 4 and Example 4a**

| API particle size | Example 4 | Example 4a |
|---|---|---|
| D50 | 9.18µm | 31µm |
| D90 | 22.0µm | 76µm |

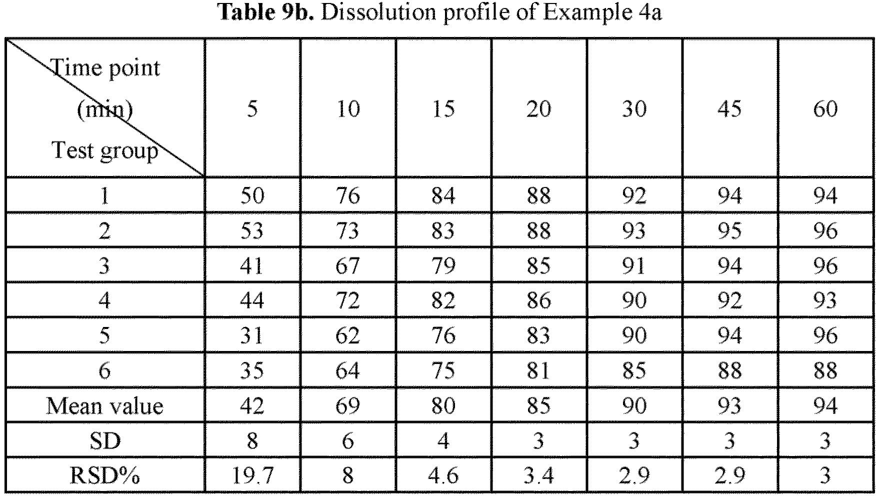

Generally speaking, for poorly soluble drugs, the particle size of the APIs is often a key factor to be considered in formulation development. Drugs with different particle sizes often show faster in vitro dissolution and better in vivo absorption behavior of APIs with small particle sizes due to their differences in specific surface area. Surprisingly, however, Example 4 and Example 4a exhibited similar dissolution behaviors, and the similarity factor f2 of in vitro dissolution was 52 (if f2 was greater than 50, the dissolution was considered to be similar). The dissolution profile of Example 4a is shown in Table 9b.

**Table 9c. Comparison of pharmacokinetic parameters of APIs with different particle sizes in beagle dogs at a dose of 250 mg**

| Formulation prescription | | Example 4 | | Example 4a | |
|---|---|---|---|---|---|
| Dosage | | 750 mg/dog | | | |

| Pharmacokinetic parameters | Unit | Mean value | SD | Mean value | SD |
|---|---|---|---|---|---|
| Cₘₐₓ | ng/mL | 33400 | 2090 | 29900 | 2010 |
| AUC₀₋ₜ | ng*h/mL | 181000 | 41000 | 155000 | 26100 |
| AUC_{0-t_dose} | h*kg*ng/mL/mg | 1990 | 428 | 1790 | 290 |

The pharmacokinetic data in beagle dogs further reached a similar conclusion: AN0025 API with different particle sizes had no effect on product quality. The tablet provided by the invention has better durability to APIs of different particle sizes. The in vivo pharmacokinetic parameters of Example 4 and Example 4a are compared in Table 9c. The drug concenration-time curve of Example 4a in beagle dogs at a dose of 750 mg/dog is shown in Figure 7.

### 10. Example 5

The tablet of Example 5 of the present invention was prepared according to the ingredients and contents as described in Table 9a.

**Table 10a. Prescription component of Example 5**

| Ingredient name | weight percentage %W/W | Theoretical prescription volume (mg) |
|---|---|---|
| Granulation part | | |
| Compound AN0025 | 50 | 250 |
| mannitol | 5.63 | 28.15 |
| low-substituted hydroxypropyl cellulose | 16 | 80 |
| hydroxypropyl cellulose | 4 | 20 |
| carboxymethylcellulose calcium | 5 | 25 |
| microcrystalline cellulose | 16.88 | 84.4 |
| sodium stearyl fumarate | 1 | 5 |

| Additional part | | |
|---|---|---|
| sodium stearyl fumarate | 1.5 | 7.5 |

| Coating | | |
|---|---|---|
| Opadry^{®} | coating weight gain 4% | 20 |

The preparation process is as follows:

### Step 1 Mixing

The weighed Compound AN0025, Mannitol, low-substituted hydroxypropyl cellulose, hydroxypropyl cellulose, Carboxymethylcellulose calcium, microcrystalline cellulose were added to hopper mixer and mixed evenly.

### Step 2 Lubricating I

Use a hopper mixer to mix the sodium stearyl fumarate in the granulation part with the mixture in step 1 until uniform.

### Step 3 Granulating

Use a roller compactor to press the mixture into a long strip, and the rolling parameters are: compaction force 7Mpa; roll speed: 2rpm; gap width: 1.5mm.

### Step 4 Sizing

The long strips are crushed and sieved through a 0.8 mm screen to obtain granules with an internal phase.

### Step 5 Lubricating II

The granules prepared above were mixed with an additional part of sodium stearyl fumarate.

### Step 6 Tabletting

At the desired target tablet weight of 500 mg, use a tablet press to compress into tablets of the appropriate shape.

### Step 7 Coating

Fully disperse Opadry in purified water, prepare a coating solution, and perform film coating through a coating machine with a target coating weight gain of 4%.
The dissolution data are shown in Table 9b, and the dissolution RSD of all Time points is less than 10%.

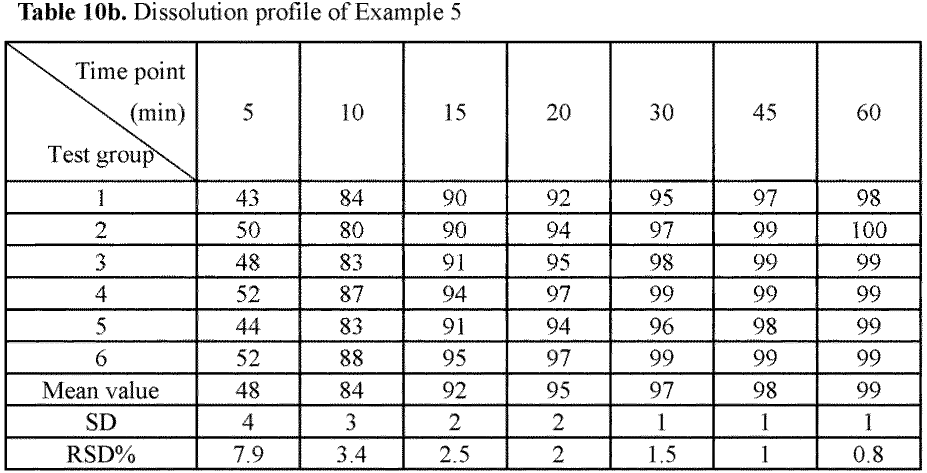

## Claims

1. A solid pharmaceutical composition, comprising the compound described in formula 1 or a pharmaceutically acceptable salt thereof and a pharmaceutically acceptable carrier, wherein the compound described in formula 1 or a pharmaceutically acceptable salt thereof comprises from about 30% to about 80% of the total weight of the solid pharmaceutical composition; the dosage form of the solid pharmaceutical composition is a tablet.

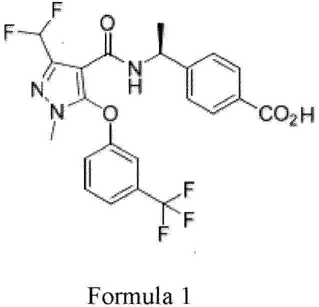

2. The solid pharmaceutical composition of claim 1, wherein the compound of formula 1 or a pharmaceutically acceptable salt thereof comprises from about 40% to about 60% of the total weight of the solid pharmaceutical composition.

3. The solid pharmaceutical composition according to any one of the preceding claims, wherein the pharmaceutically acceptable carrier comprises one or more selected from fillers, binders, lubricants, disintegrants and glidants.

4. The solid pharmaceutical composition according to any one of the preceding claims, wherein the pharmaceutically acceptable carriers include:
a. a filler, which comprises from about 5% to about 60% of the total weight of the solid pharmaceutical composition;
b. a binder, which comprises from about 0% to about 8% of the total weight of the solid pharmaceutical composition;
c. a disintegrant, which comprises from about 0% to about 30% of the total weight of the solid pharmaceutical composition;
d. a lubricant, which comprises from about 0.1% to about 10% of the total weight of the solid pharmaceutical composition;
e. a glidant, which comprises from about 0% to about 5% of the total weight of the solid pharmaceutical composition.

5. The solid pharmaceutical composition according to any one of the preceding claims, wherein the pharmaceutically acceptable carrier comprises:
a. a filler, which comprises from about 10% to about 50% of the total weight of the solid pharmaceutical composition;
b. a binder, which comprises from about 2% to about 6% of the total weight of the solid pharmaceutical composition;
c. a disintegrant, which comprises from about 0.1% to about 25% of the total weight of the solid pharmaceutical composition;
d. a lubricant, which comprises from about 0.5% to about 8% of the total weight of the solid pharmaceutical composition;
e. a glidant, which comprises from about 0% to about 3% of the total weight of the solid pharmaceutical composition.

6. The solid pharmaceutical composition according to any one of the preceding claims, wherein the pharmaceutically acceptable carrier comprises:
a. a filler, which comprises from about 20% to about 40% of the total weight of the solid pharmaceutical composition;
b. a binder, which comprises from about 3% to about 5% of the total weight of the solid pharmaceutical composition
c. a disintegrant, which comprises from about 1% to about 25% of the total weight of the solid pharmaceutical composition;
d. a lubricant, which comprises from about 1% to about 6% of the total weight of the solid pharmaceutical composition;
e. a glidant, which comprises from about 0% to about 1% of the total weight of the solid pharmaceutical composition.

7. The solid pharmaceutical composition of any one of the preceding claims, wherein,
the filler is selected from microcrystalline cellulose, calcium phosphate, calcium hydrogen phosphate, starch, dextrin, pregelatinized starch, mannitol, sorbitol, lactose, dextran, sucrose, dextrose and combinations thereof;
The binder is selected from cellulose ethers, povidone, copovidone, starch, corn syrup and combinations thereof; wherein the cellulose ethers include but are not limited to hypromellose, hydroxypropyl cellulose, methyl cellulose, hydroxyethyl cellulose;
the disintegrant is selected from sodium croscarmellose, crospovidone, sodium carboxymethyl starch, carboxymethylcellulose calcium, low-substituted hydroxypropyl cellulose and combinations thereof; and
the lubricant is selected from magnesium stearate, sodium stearyl fumarate, polyethylene glycol, glycerin, behenate, talc and combinations thereof.

8. A solid pharmaceutical composition according to any one of the preceding claims, wherein, the filler is selected from microcrystalline cellulose, lactose, mannitol, sorbitol and combinations thereof;
the binder is selected from hydroxypropyl cellulose, hypromellose, povidone, copovidone and combinations thereof;
the disintegrant is selected from sodium croscarmellose, crospovidone, sodium carboxymethyl starch, carboxymethylcellulose calcium, low-substituted hydroxypropyl cellulose and combinations thereof; and
the lubricant is selected from magnesium stearate, sodium stearyl fumarate and combinations thereof.

9. The solid pharmaceutical composition according to any one of the preceding claims, comprising granules having an internal phase.

10. The solid pharmaceutical composition of claim 9, further including an external phase adjacent to the granules having an internal phase.

11. The solid pharmaceutical composition of claim 9 or 10, wherein the granules having an internal phase are prepared by a dry preparation process.

12. The solid pharmaceutical composition of claim 9 or 10, wherein the granules having an internal phase are prepared by a wet preparation process.

13. The solid pharmaceutical composition according to any one of the preceding claims, wherein the compound of formula 1 has a particle size with a D90 of less than about 200 microns.

14. The solid pharmaceutical composition according to any one of the preceding claims, wherein the compound of formula 1 has a particle size with a D90 of less than about 100 microns.

15. The solid pharmaceutical composition of any one of the preceding claims, further comprising a coating.

16. The solid pharmaceutical composition according to any one of the preceding claims, comprising from about 50 mg to about 500 mg of the compound of formula 1.

17. The solid pharmaceutical composition according to any one of the preceding claims, comprising from about 100 mg to about 300 mg of the compound of formula 1.

18. Use of the solid pharmaceutical composition of any one of the preceding claims for the manufacture of a medicament for the treatment of multiple sclerosis, rheumatoid arthritis, or cancer.

19. The use of claim 18, wherein the cancer comprises: skin cancer, breast cancer, colorectal cancer, prostate cancer, kidney cancer, ovarian cancer, cervical cancer, endometrial cancer, glioblastoma tumor, lung cancer, head and neck cancer, medulloblastoma, and urethral cancer.
